(19) Europäisches Patentamt
European Patent Office
Office européen des brevets

(11) **EP 3 449 239 B1**

(12) **FASCICULE DE BREVET EUROPEEN**

(45) Date de publication et mention de la délivrance du brevet:
**16.08.2023 Bulletin 2023/33**

(21) Numéro de dépôt: **17725314.3**

(22) Date de dépôt: **26.04.2017**

(51) Classification Internationale des Brevets (IPC):
**G01N 21/25** *(2006.01)*    **G01N 21/31** *(2006.01)*
**G01N 21/35** *(2014.01)*    **G01N 21/3554** *(2014.01)*
**G01N 21/47** *(2006.01)*    **A61B 5/00** *(2006.01)*
**G01N 21/359** *(2014.01)*    **G01N 21/3563** *(2014.01)*

(52) Classification Coopérative des Brevets (CPC):
**G01N 21/314; A61B 5/1455; A61B 5/443;**
**A61B 5/4875; A61B 5/4881; G01N 21/256;**
**G01N 21/3554; G01N 21/3563; G01N 21/359;**
**G01N 21/4785;** G01N 2021/1734;
G01N 2021/1782; G01N 2021/215;
G01N 2021/3133; G01N 2021/4709;     (Cont.)

(86) Numéro de dépôt international:
**PCT/FR2017/050992**

(87) Numéro de publication internationale:
**WO 2017/187088 (02.11.2017 Gazette 2017/44)**

(54) **PROCÉDÉ ET APPAREIL DE MESURE DE LA CONCENTRATION EN EAU DANS UN MATÉRIAU DIFFUSANT LA LUMIÈRE.**

VERFAHREN UND VORRICHTUNG ZUR MESSUNG DER WASSERKONZENTRATION IN EINEM LICHTSTREUENDEN MATERIAL

METHOD AND APPARATUS FOR MEASURING THE WATER CONCENTRATION IN A LIGHT-DIFFUSING MATERIAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.04.2016 FR 1653740**

(43) Date de publication de la demande:
**06.03.2019 Bulletin 2019/10**

(73) Titulaire: **Connected Physics**
**92220 Bagneux (FR)**

(72) Inventeurs:
• **NAPPEZ, Thomas**
**92170 Vanves (FR)**
• **LEPAGE, Hadrien**
**75010 Paris (FR)**

(74) Mandataire: **Touroude & Associates**
**2, bis rue Alfred Nobel**
**77420 Marne la Vallée (FR)**

(56) Documents cités:
**US-A1- 2004 230 106    US-A1- 2011 184 683**
**US-A1- 2013 210 058    US-A1- 2014 016 132**
**US-B1- 6 281 498    US-B2- 9 259 486**

• **STEFAN ANDREE ET AL: "Feasibility of dermal water content determination by spatially resolved reflectance", MEDICAL LASER APPLICATION, ELSEVIER, NL, vol. 26, no. 3, 15 mai 2011 (2011-05-15), pages 109-118, XP028248370, ISSN: 1615-1615, DOI: 10.1016/J.MLA.2011.05.003 [extrait le 2011-06-24]**

EP 3 449 239 B1

• **RAMI NACHABÉ ET AL: "Estimation of lipid and water concentrations in scattering media with diffuse optical spectroscopy from 900 to 1600 nm", INTERNATIONAL SOCIETY FOR OPTICAL ENGINEERING, vol. 15, no. 3, 1 janvier 2010 (2010-01-01), page 037015, XP055329702, PO Box 10 Bellingham WA 98227-0010 USA ISSN: 1083-3668, DOI: 10.1117/1.3454392**

(52) Classification Coopérative des Brevets (CPC):
(Cont.)
G01N 2021/4735

**Description**

Domaine de l'invention

La présente invention concerne le domaine de la mesure de la concentration de l'eau contenue dans un matériau, et plus particulièrement dans un matériau diffusant la lumière telle que la peau humaine, les végétaux, les papiers, les plâtres, etc.

D'une manière générale, on connaît dans l'état de l'art différentes familles de mesures de l'hydratation d'un matériau.

Une première famille concerne des mesures indirectes, basées sur la relation entre la teneur en eau et la conductivité électrique, qui ne sont applicables uniquement si l'on considère que la conductivité électrique mesurée est une image de la concentration en eau, eau qui permet de plus ou moins diluer des particules chargées électriquement, particules qui sont initialement présentes dans le matériau à analyser et responsables de sa conduction électrique.

Pour la mesure de l'hydratation de la peau, on utilise par exemple des cornéomètres dont la mesure est basée sur les propriétés électriques de la peau. Un courant passe entre deux électrodes fixées à la peau. Plus la couche cornée est hydratée, plus le courant électrique passe bien. Des facteurs peuvent influencer les mesures : sueur, température de la peau, taux d'humidité du milieu ambiant, état de la surface cutanée... (un état rugueux réduit la surface de contact entre la sonde et la peau, l'application d'un topique riche en eau et en substances ioniques peut modifier la mesure).

La deuxième famille de mesures est basée sur une analyse directe par spectroscopie optique élastique ou inélastique.

Etat de la technique

La présente invention s'inscrit dans cette deuxième famille de mesure directe. L'évaluation de la quantité d'eau présente dans de la matière organique et dans des tissus biologiques est réalisée par spectrophotométrie infrarouge. Cette technique utilise une lumière composée de plusieurs longueurs d'onde pour obtenir le spectre de réflectance ou de transmission de la matière à analyser.

On obtient ainsi une évaluation de la concentration en eau dans l'échantillon en comparant la puissance reçue à une longueur d'onde proche d'une bande d'absorption des molécules d'eau à celle reçue à une longueur d'onde éloignée des bandes d'absorption des molécules d'eau.

Plusieurs modes de réalisation de cette technique de mesure existent :

- Emission de lumière blanche sur l'échantillon et récupération dans un spectromètre qui permet d'étudier le spectre de l'échantillon de manière résolue,

- Emission de lumière monochromatique avec des émetteurs différents utilisés séquentiellement ce qui permet d'utiliser un photodétecteur à la place d'un spectromètre,

- ces techniques peuvent être réalisées en optique de volume ou en optique fibrée.

On connaît par exemple le brevet américain US 8180419 décrivant un procédé de détermination de l'hydratation tissulaire, comprenant une étape de détection d'un rayonnement électromagnétique diffusé et réfléchi par le tissu. Ce rayonnement électromagnétique détecté est utilisé pour déterminer la largeur de bande d'absorption spectrale par la corrélation de la largeur de bande d'absorption spectrale d'un indice d'hydratation des tissus.

On connaît aussi par la demande de brevet européen EP 2956049 un procédé pour la détermination non invasive de l'hydratation, l'état d'hydratation, l'eau corporelle totale, ou la concentration d'eau par spectroscopie quantitative.

Cette solution comprend des sous-systèmes, optimisés pour se conformer aux complexités de la spectroscopie tissulaire, aux exigences de rapport signal sur bruit et de précision photométrique élevés, aux erreurs d'échantillonnage de tissu, à la maintenance d'étalonnage et au transfert d'étalonnage. Les sous-systèmes comprennent un sous-système d'illumination, un sous-système d'échantillonnage de tissu, un sous-système de spectromètre, un sous-système d'acquisition de données, un sous-système informatique, et un sous-système d'étalonnage.

Le brevet américain US 6442408 décrit une autre solution pour quantifier de manière non invasive l'hydratation de la couche cornée d'un sujet vivant. Des mesures spectrales dans le proche infrarouge (NIR) sont effectuées et les spectres d'absorption NIR obtenus sont transmis à un analyseur pour un traitement ultérieur. Celui-ci consiste à détecter et éliminer les mesures spectrales non valides, et à augmenter le rapport signal sur bruit. Un modèle d'étalonnage mis au point à partir d'un exemple d'ensemble de mesures est appliqué pour prédire l'hydratation de l'échantillon.

La méthode de mesure d'hydratation fournit des informations supplémentaires sur la variabilité tissulaire systématique, à savoir la teneur en eau de la couche épidermique de la peau et la profondeur de la lumière incidente de pénétration.

Le document US2014/0016132 A1 décrit un procédé de mesure de la concentration en eau dans un matériau diffusant utilisant des sources de lumière et des capteurs associés pour mesurer la diffusion et l'absorption dans le matériau et utilisant des simulation numériques pour déterminer la concentration en eau. Le document US2011/018468 A1 décrit un procédé similaire en utilisant des échantillons de référence pour déterminer la concentration en eau. Les documents S.Andree et Al., Medical Laser application (2011), vol.26, pp.109-118 et R.Nachabé et Al., Journal of Biomédical Optics, vol.15, issue 3, May/June 2010, pp.037015-1-037015-10 décrivent un procédé similaire utilisant une source lumineuse et un capteur ainsi que des échantillons de calibration représentatifs du milieu à étudier pour déterminer la concentration en eau.

## Inconvénients de l'art antérieur

Ces solutions basées sur la mesure directe par analyse spectroscopique ne sont pas totalement satisfaisantes car l'intensité de la lumière mesurée est fonction en partie seulement de l'absorption des molécules d'eau dans le milieu analysé, la peau par exemple. Des facteurs perturbateurs interviennent et ne permettent pas une déduction directe de la mesure spectroscopique de la teneur en eau.

Aucune de ces techniques de mesure de l'art antérieur ne permet d'obtenir de valeurs quantitatives et absolues de la concentration en eau.

En effet, elles reposent sur plusieurs hypothèses :

- l'absorption par l'eau du rayonnement émis à la (aux) longueur(s) d'ondes éloignée(s) des bandes d'absorption est négligeable,
- le coefficient de diffusion est identique et/ou son impact est négligeable pour l'ensemble des longueurs d'onde de travail,
- la profondeur de pénétration du rayonnement dans l'échantillon est identique pour l'ensemble des longueurs d'onde de travail et, le cas échéant, l'ensemble des différents émetteurs.

Plusieurs solutions de l'art antérieur proposent de remédier à ce problème en enregistrant une table d'étalonnage permettant de fournir une information retraitée en fonction des données résultant de mesures sur des échantillons de référence.

Cette solution est très lourde à mettre en oeuvre et ne permet de procéder à une mesure que sur des matériaux préalablement caractérisés, et dans les mêmes conditions que celles utilisées lors de cette phase de calibration.

## Solution apportée par l'invention

Afin de remédier à ces inconvénients, la présente invention concerne selon son acception la plus générale un procédé de mesure de la concentration de l'eau dans un matériau diffusant la lumière tel que défini dans la revendication 1.

De préférence, la longueur d'onde Lambda$_i$ est choisie autour de 1450, de 1940, de 970 ou de 1190 nanomètres, pour les sources lumineuses et capteurs délivrant un signal représentatif de la diffusion dans une longueur d'onde correspondant à un pic d'absorption de l'eau.

Selon une variante, la longueur d'onde Lambda$_i$ est choisie autour de 1050, de 1070, de 1100 ou de 1300 nanomètres, pour les sources lumineuses et capteurs délivrant le signal représentatif de la diffusion dans une longueur d'onde différent d'un pic absorption de l'eau.

L'information numérique est fournie par une ou des fonctions dont la détermination est réalisée par une étape de calibration par acquisition des signaux Sj pour des échantillons dont la teneur en eau est connue.

L'invention concerne aussi un équipement de mesure de la concentration de l'eau dans un matériau diffusant la lumière tel que défini dans la revendication 4. L'information numérique est idéalement fournie par une ou des fonctions dont la détermination est réalisée par une étape de calibration par acquisition des signaux Sj pour des échantillons dont la teneur en eau est connue.

Avantageusement, lesdites N sources lumineuses SL$_i$ et lesdits M capteurs P$_j$ sont logées dans support s'ouvrant par une fenêtre de contact avec le matériau à caractériser, ledit support comportant un écran empêchant la transmission directe et/ou réfléchie, sans pénétration préalable dans ledit matériau diffusant, de la lumière depuis une source lumineuse vers un capteur.

De préférence, la fenêtre du support présente un traitement anti-reflet.

Selon un mode de réalisation préféré, lesdites N sources lumineuses SL$_i$ sont polarisées et la direction du faisceau émis forme un angle de Brewster avec l'axe optique desdits M capteurs P$_j$.

Selon un mode de réalisation particulier, l'équipement de mesure de la concentration de l'eau dans un matériau diffusant comprend des moyens de radiocommunication pour transmettre périodiquement le résultat de la mesure à un équipement distant.

Selon une variante, les sources de lumière, les capteurs et le circuit électronique de l'équipement constituent un patch présentant des moyens de liaison réversible sur un patient.

L'équipement selon l'invention peut par ailleurs présenter les caractéristiques suivantes, indépendamment ou en combinaison :

- il comprend au moins deux couples source lumineuse/capteur, un premier couple source lumineuse/capteur définissant entre le capteur et la source une première distance de séparation, et un deuxième couple source lumineuse/capteur définissant une deuxième distance de séparation

- les sources des deux couples émettent toutes deux avec un spectre centré autour d'une longueur d'onde proche d'un pic d'absorption de l'eau ou en ce que l'une des deux sources émet avec un spectre centré autour d'une longueur d'onde proche d'un pic d'absorption de l'eau et l'autre source, avec un spectre centré autour d'une longueur d'onde d'absence d'absorption de l'eau

- les sources des deux couples émettent toutes deux avec un spectre centré autour d'une longueur d'onde d'absorption de 1450 nm, 1500 nm ou 1550 nm

- les sources des deux couples émettent toutes deux dans une longueur d'onde d'absence d'absorption de l'eau

- les sources des deux couples émettent toutes deux avec un spectre centré autour de 1300 nm

- l'équipement comprend un troisième couple source lumineuse/capteur, la source lumineuse émettant dans une longueur d'onde d'absorption de l'eau

- l'équipement comprend un troisième couple source lumineuse/capteur, la source lumineuse émettant dans une longueur d'onde d'absence d'absorption de l'eau

- la source lumineuse émettant dans une longueur d'onde d'absorption de l'eau est disposée au plus proche du capteur

- l'équipement comprend un capteur et trois sources, les trois sources appartenant respectivement à trois couples source/capteur distincts, ou comprenant deux capteurs et deux sources, l'une des deux sources émettant dans une longueur d'onde proche d'un pic d'absorption de l'eau, et l'autre source dans une longueur d'onde d'absence d'absorption de l'eau

- l'équipement comprend entre les deux sources distinctes une série de capteurs distincts disposés en ligne

- l'équipement comprend entre les deux sources une barrette linéaire de photodiodes

- l'équipement comprend un capteur central ou excentré et autour de ce capteur une succession de sources d'émission lumineuse centrées autour de longueurs d'ondes d'absorption et/ou d'absence d'absorption de la lumière

- l'équipement comprend au moins une source d'émission lumineuse centrale ou excentrée émettant avec un spectre de lumière centré autour d'une longueur d'ondes d'absorption ou d'absence d'absorption de la lumière et autour de cette source, une succession de capteurs

- l'équipement comprend un masque opaque aux différentes longueurs d'ondes employées s'interposant physiquement entre une source et un capteur adjacent

- le masque forme autour de chaque source un tunnel de concentration en une zone précise de la lumière émise

L'invention concerne également des applications du procédé pour :

- la caractérisation de l'hydratation de la peau.

- la caractérisation de l'hydratation d'une mèche de cheveux.

- la caractérisation de la concentration d'eau dans une matrice cellulosique [papier ; carton ; bois].

- la caractérisation de la concentration d'eau dans une matrice alimentaire.

- la caractérisation de la concentration d'eau dans un végétal.

- la caractérisation de la concentration d'eau dans un hydrocarbure.

- la caractérisation de la concentration d'eau dans un produit chimique ou une poudre.

- la caractérisation de la concentration d'eau dans un produit médicamenteux.

- la caractérisation de la concentration d'eau dans un matériau de construction.

- la caractérisation de la concentration d'eau dans un revêtement d'un bâtiment.

- la caractérisation de la concentration d'eau dans un textile.

- la caractérisation de la concentration d'eau dans le cuir.

- la caractérisation de l'hydratation de la peau et/ou la caractérisation de la qualité et/ou la quantité de collagène ou de la kératine de la peau.

Description détaillée d'un exemple non limitatif de l'invention

La présente invention sera mieux comprise à la lecture de la description détaillée d'un exemple non limitatif de l'invention qui suit, se référant aux dessins annexés où :

- la figure 1 représente une vue schématique d'un équipement conforme à l'invention
- la figure 2 représente une vue schématique de l'architecture fonctionnelle.
- la figure 3 représente une vue schématique du principe de calcul de la concentration d'eau.

- la figure 4 représente une coupe dans l'échantillon schématisant le trajet de rayons lumineux entre une source et un capteur.
- la figure 5 est issue des simulations de l'interaction lumière-échantillon et indique le logarithme du signal log(S) reçu dans le capteur en fonction du coefficient d'absorption = c * $\mu_a$ de l'échantillon et son coefficient de diffusion réduit $\mu_s$'.
- la figure 6 représente une vue schématique d'une première variante de mode de réalisation.
- la figure 7 représente une vue schématique d'une deuxième variante de mode de réalisation.
- la figure 8 représente une vue schématique d'une troisième variante de mode de réalisation.
- les figures 9 et 10 représentent par des vues en perspective, deux variantes de réalisation de masques pour le dispositif selon l'invention.
- la figure 11 est issue des simulations de l'interaction lumière-échantillon et indique le logarithme du signal log(S) reçu dans le capteur en fonction du coefficient d'absorption = c * $\mu_a$ de l'échantillon et son coefficient de diffusion réduit $\mu_s$' pour deux différents couples émetteurs capteurs.
- la figure 12 représente le spectre théorique d'absorption de l'eau, et les spectres obtenus suite à la traversée de la lumière de différentes sources à travers un échantillon absorbant et diffusant la lumière, ces sources ayant des pics d'émission centrés respectivement autour de 1450 nm, 1500 nm et 1550 nm.
- la figure 13 représente une vue schématique d'une variante non-couverte par les revendications du principe de calcul de la concentration d'eau à partir de simulations numériques. Les fonctions analytiques $f_i$ sont obtenues en résolvant l'équation de transfert radiatif, par des simulations numériques Monte-Carlo par exemple. Ces simulations modélisent l'interaction entre la lumière et l'échantillon et prennent en compte les paramètres opto-géométriques.

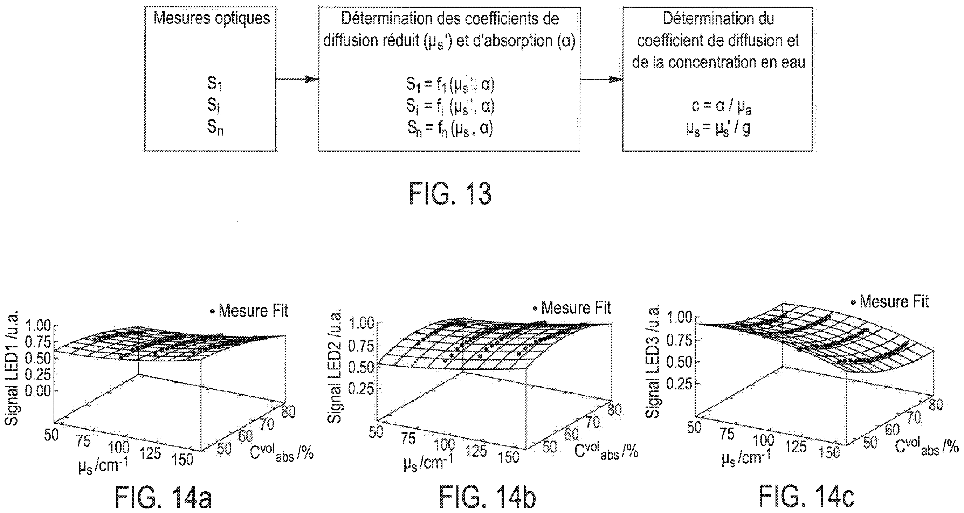

- les figures 14a à 14c représentent trois fonctions analytiques obtenues respectivement pour trois sources distinctes, représentant les signaux perçus après traversée de la lumière de ces trois sources, par différents échantillons fantômes de concentration en eau et de coefficient de diffusion connus. On constate que ces fonctions prennent la forme de surfaces courbes. Un échantillon inconnu analysé par le dispositif selon l'invention pourvu de trois sources, permettra la détection de trois signaux de valeurs numériques S1, S2, S3. A ces trois signaux correspondent sur les représentations des fonctions correspondantes, des zones ou des isocontours et donc des couples (concentration en eau, coefficient de diffusion). La recherche de la concentration en eau et du coefficient de diffusion de l'échantillon inconnu, revient à identifier s'il existe une solution unique pour le couple (concentration en eau, coefficient de diffusion) satisfaisant à la fois les trois isocontours correspondants sur les trois fonctions,

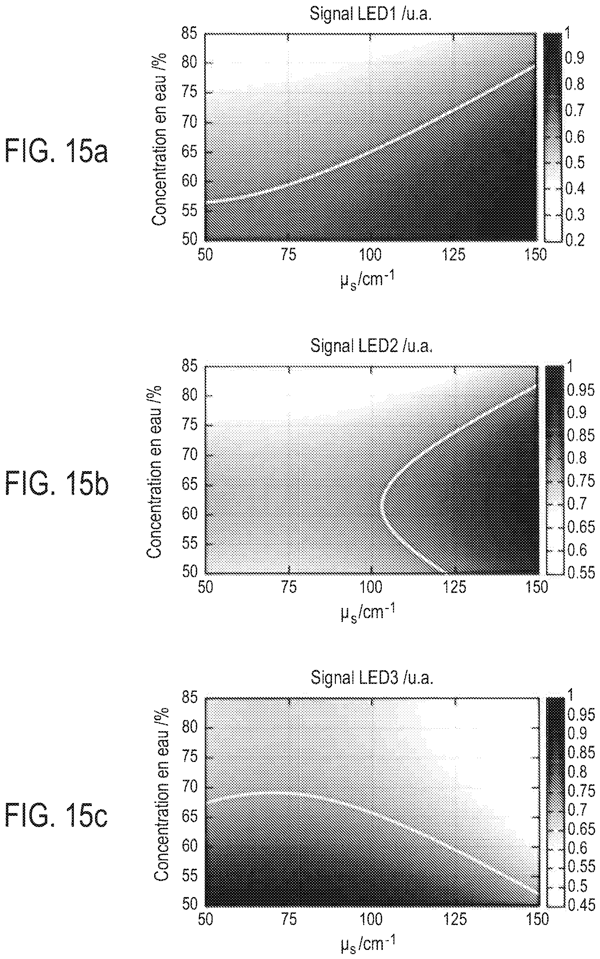

- les figures 15a à 15c illustrent une autre représentation possible de ces trois fonctions analytiques, sur chacune desquelles est en outre repéré par une courbe, l'ensemble des couples (concentration en eau, coefficient de diffusion) d'échantillons donnant le même signal S1 pour la courbe de la figure 15a, S2 pour la courbe de la figure 15b, S3 pour la courbe de la figure 15c, et à partir desquelles sera déduit le couple unique (concentration en eau, coefficient de diffusion) retrouvé sur les trois courbes. A titre d'exemple de calcul du bon couple (concentration en eau, coefficient de diffusion), les valeurs normalisées pour ces trois signaux sont : S1 = 0,59 ; S2 = 0,78 ; S3 = 0,71
- la figure 16 représente avec cet exemple un tableau comparatif des résultats de concentration en eau et de coefficient de diffusion obtenus selon le procédé de l'invention (« Mesure ») sur un échantillon connu, vis à vis des concentrations en eau et coefficient de diffusion connus de cet échantillon (« Expérimental »)
- la figure 17 illustre pour ce même exemple la méthode de minimisation par moindre carré pour le calcul du bon couple (concentration en eau, coefficient de diffusion). Cette figure représente la variance pour les trois signaux S1, S2 et S3 qui est bien minimale au couple recherché (concentration en eau = 66% et coefficient de diffusion = 119 /cm).
- les graphiques des figures 18a et 18b illustrent les corrélations linéaires observées respectivement entre la concentration en eau et le coefficient de diffusion mesurés selon le procédé de l'invention, et les valeurs correspondantes expérimentales d'échantillons connus
- la figure 19 représente deux courbes illustrant les évolutions de la concentration en eau mesurée par le procédé selon l'invention durant 24 heures, de deux échantillons similaires de peau d'un même individu (cochon) respectivement laissée séchée à l'air libre (courbe « air ») et en contact avec du liquide physiologique (courbe « eau »), montrant une évolution logique : les mesures obtenues pour l'échantillon en contact avec du liquide physiologiques sont relativement constantes, et celles de l'échantillon laissé à l'air libre diminuent avec la durée d'exposition à l'air
- les figures 20a et 20b représentent deux histogrammes illustrant les évolutions dans le temps des mesures de la concentration en eau selon le procédé de l'invention, effectuées sur une personne volontaire (derme du mollet) respectivement lors d'une journée sans activité sportive particulière (figure 20a) et lors d'une journée interrompue par une séance de sport à une heure précise (16h30) sans que le volontaire ne boive ensuite jusqu'à 21h30 puis dîne,
- les figures 21a à 21g représentent différents modes de réalisation du dispositif de mise en oeuvre du procédé selon l'invention

- la figure 22 représente une vue schématique du principe de calcul de la concentration d'eau selon l'invention à partir d'échantillons fantôme, dans lequel les fonctions analytiques $f_i$ sont obtenues à partir d'échantillons fantôme reproduisant le comportement optique de la peau.

L'exemple de réalisation de l'invention décrit dans ce qui suit à titre d'exemple non limitatif permet d'obtenir une mesure de la concentration en eau dans un échantillon diffusant la lumière de manière absolue et non relative, sans utilisation d'un spectromètre.

En effet, la spectrométrie classique est une technique versatile qui n'est pas adaptée aux particularités de la mesure absolue dans un échantillon diffusant la lumière. La mesure du coefficient de diffusion de la lumière dans la matière organique et les tissus biologiques à la longueur d'onde de travail doit en effet être effectuée pour dissocier la contribution de la diffusion de celle de l'absorption dans le signal reçu.

Pour cela, il est ici proposé d'utiliser la spectroscopie résolue spatialement (SRS pour Spatially Resolved Spectroscopy) et de la coupler avec une étape de calibration expérimentale ou de modélisation physique de l'interaction entre la lumière et l'échantillon (cette dernière variante n'étant pas couverte par les revendications). Ces modélisations reproduisent les paramètres optogéométiques ainsi que l'échantillon à analyser et résolvent l'équation de transfert radiatif (RTE pour Radiative Transfer Equation) grâce à des algorithmes Monte Carlo ou à l'approximation de Kubelka-Munk par exemple.

En fonction des puissances émises par chaque source et reçues par chaque capteur, cette calibration ou cette modélisation permet de remonter au coefficient de diffusion réduit et au coefficient d'absorption de l'échantillon. La longueur d'onde de la lumière émise étant proche et autour d'une bande d'absorption de l'eau correspondant, ce coefficient d'absorption permet de connaître la concentration en eau dans l'échantillon.

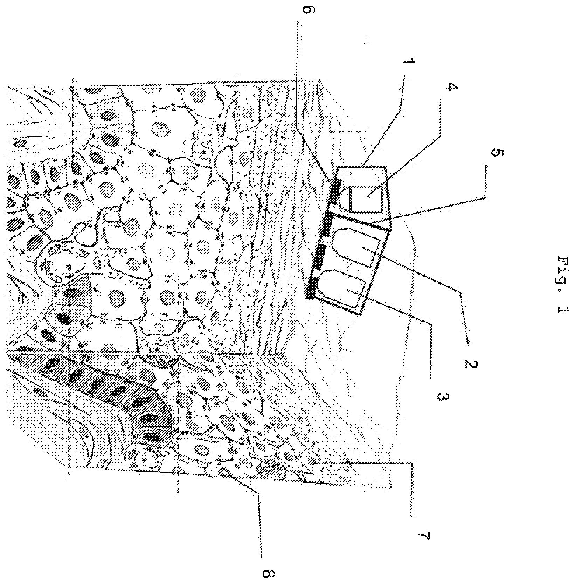

La figure 1 représente une vue simplifiée d'un équipement selon l'invention. Il est constitué par un boîtier (1) contenant deux diodes électroluminescentes (2, 3) et une photodiode (4), alignés dans l'exemple décrit, et écartés de quelques dizaines de micromètres à quelques millimètres. Les diodes électroluminescentes (2, 3) sont séparées de la photodiode (4) par une paroi opaque (5) empêchant la transmission directe de la lumière.

Le boîtier (1) présente une fenêtre fermée par une lame transparente (6) éventuellement revêtue par filtre anti-reflet, dont la surface peut être appliquée directement contre la couche granuleuse (7) de la peau (8).

Le boitier (1) comprend aussi un calculateur et des moyens de communication et/ou un moyen d'affichage.

Les sources (2, 3) utilisés pour réaliser la mesure sont préférentiellement des diodes électroluminescentes (recouvertes ou non d'une lentille pour orienter le rayonnement) ou des lasers, plus particulièrement des lasers à semiconducteurs (aussi appelés diodes laser). Les longueurs d'ondes employées peuvent être situées à proximité des valeurs suivantes : 970nm, 1050nm, 1190nm, 1300nm, 1450nm et 1940nm.

Le capteur (4) utilisé est préférentiellement une photodiode, pouvant être utilisée dans le mode photovoltaïque ou photoconducteur. Il peut également être un capteur à pixels (CMOS, CCD), une photodiode à avalanche, un photomultiplicateur, un phototransistor, une photorésistance...

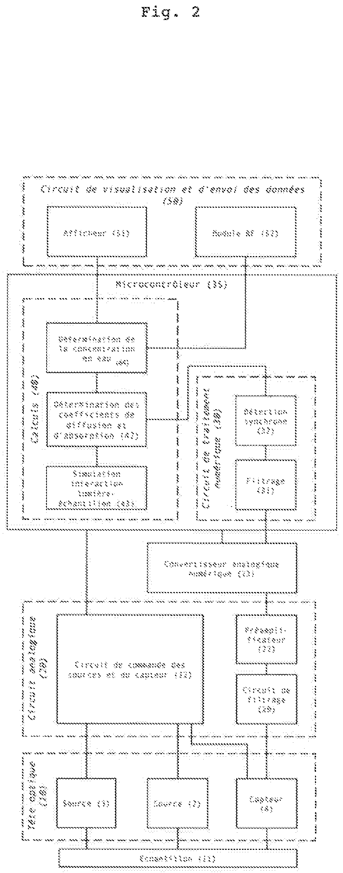

La figure 2 illustre l'architecture fonctionnelle de l'équipement.

Il comprend une tête optique (10) appliquée contre l'échantillon (11) pour transmettre la lumière venant des deux sources (2, 3) et recevoir la lumière retransmise par l'échantillon (11) par un capteur (4).

Un circuit de commande (12) des sources (2, 3) et du capteur (4) alimente la tête optique (10) en assurant l'allumage séquentiel des sources (2, 3). Dans l'exemple décrit, l'alimentation est modulée par un signal périodique (rectangulaire ou sinusoïdal par exemple) pour faciliter le filtrage et l'amélioration du rapport signal sur bruit.

Le signal délivré par le capteur (4) est traité par le circuit analogique (20) comprenant un circuit de filtrage (21) et un préamplificateur (22).

Le signal est ensuite numérisé par un convertisseur analogique-numérique (23). Un microcontrôleur (35) assure des fonctions de traitement du signal numérique (30) et des calculs (40).

Le calculateur commande successivement :

- une étape d'alimentation de la première diode électroluminescente (2) et la mesure de l'intensité captée par la photodiode (4) et l'enregistrement du signal $S_1$ correspondant à cette intensité puis l'extinction de la diode électroluminescente (2)

- une étape d'alimentation de la deuxième diode électroluminescente (3) et la mesure de l'intensité captée par le photodiode (4) et l'enregistrement du signal $S_2$ correspondant à cette intensité puis l'extinction de la diode électroluminescente (3)

- une étape de calcul à partir des valeurs $S_1$ et $S_2$ de l'information liée à la diffusion $I_{dif}$ et de l'information liée à l'absorption $I_{abs}$

- une étape de calcul de la concentration en eau à partir de l'information liée à l'absorption $I_{abs}$.

Le traitement de signal numérique (30) réalise un filtrage numérique (31) et une détection synchrone (32).

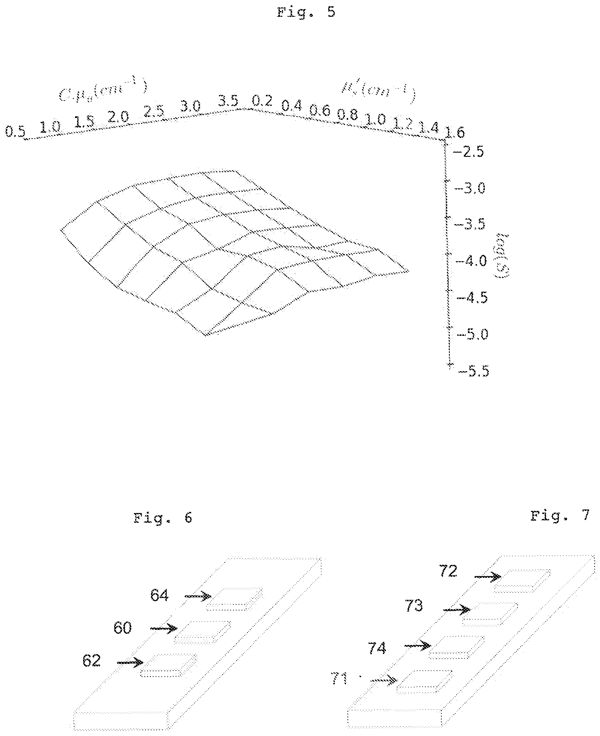

Les traitements de calcul (40) réalisent une étape de détermination des coefficients de diffusion et d'absorption (42) à partir d'abaques expérimentaux ou d'un modèle numérique (43) de simulation de l'interaction lumière-échantillon tel que ceux représentés sur les figures 5, 11, 14a, 14b, 14c, 15a, 15b ou 15c et une étape de détermination de la concentration en eau (44). Idéalement, ces coefficients sont obtenus à partir d'une information numérique fournie par une ou des fonctions dont la détermination est réalisée i) par une étape de calibration par acquisition des signaux Sj pour des échantillons dont la teneur en eau est connue ou en variante noncouverte par les revendications ii) par détermination des coefficients de diffusion et d'absorption à partir d'un modèle numérique (43) de simulation de l'interaction lumière-échantillon.

L'équipement comprend un circuit de visualisation (50) avec un afficheur (51) et un module radiofréquence (52).

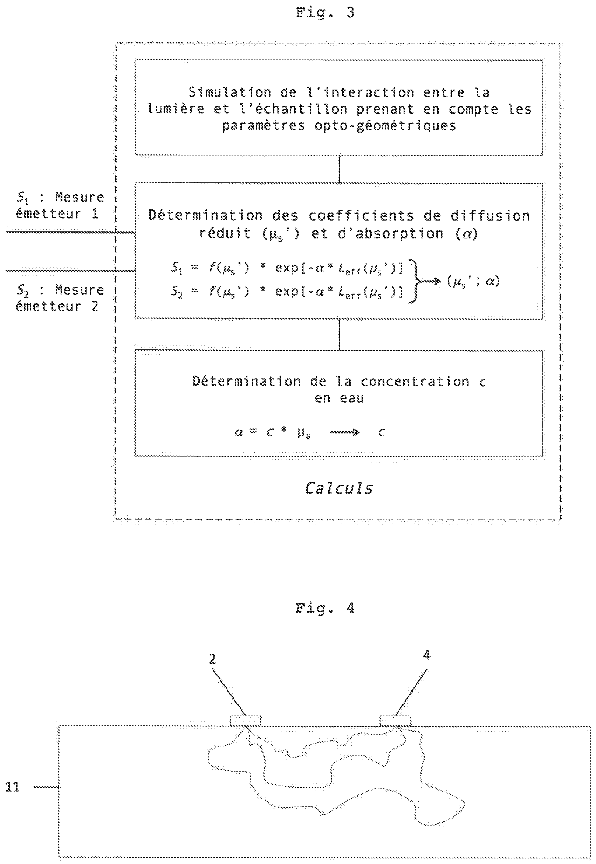

La figure 3 représente un schéma fonctionnel des calculs effectués.

Le calculateur reçoit un premier signal numérique $S_1$ correspondant à l'intensité lumineuse détectée lors de l'allumage d'une des sources (2) et un deuxième signal numérique $S_2$ correspondant à l'intensité lumineuse détectée lors de l'allumage l'autre source (3).

Ces signaux font l'objet d'un traitement pour déterminer :

- le coefficient de diffusion réduit $\mu_s'$
- le coefficient d'absorption $\alpha$

Ce traitement correspond à la résolution d'un système de deux équations à deux inconnues indiquées sur la figure 3, où les fonctions *fi* sont obtenues grâce aux abaques expérimentaux ou au modèle numérique où la fonction $f(\mu_s')$ est un préfacteur et où la fonction $L_{eff}(\mu_s')$ est la distance effective représentative du trajet parcouru par un photon dans l'échantillon avant d'atteindre le capteur.

Un modèle numérique ou analytique est enregistré en mémoire. Il correspond au résultat de la simulation de l'interaction entre la lumière et l'échantillon prenant en compte les paramètres opto-géométriques ou au résultat d'une étape de calibration expérimentale avec des échantillons dont les paramètres concentration en absorbant et coefficient de diffusion sont connus.

Dans le cas du modèle numérique, la détermination de la concentration c en eau est calculée par une fonction, qui est dans une forme simplifiée une fonction linéaire, par exemple $c = \alpha / \mu_a$ où $\mu_a$ est le coefficient d'absorption de l'eau à la longueur d'onde de travail. La détermination de cette fonction peut être réalisée par une étape de calibration par acquisition des signaux $S_1$ et $S_2$ pour des échantillons dont la teneur en eau est connue ou par le calcul de l'absorption effective de l'eau au spectre émis par les émetteurs.

D'autres modes de réalisation sont envisagés :

Dans une première variante illustrée par la figure 6, l'équipement comprend une seule source (62) présentant un rayonnement proche d'une bande d'absorption de l'eau et deux capteurs (64, 60).

Dans une deuxième variante illustrée par la figure 7, l'équipement comprend une seule source (71) présentant un rayonnement proche d'une bande d'absorption de l'eau, deux sources (72, 73) présentant un rayonnement éloigné d'une bande d'absorption de l'eau et un capteur (74).

L'emploi de différents écartements entre les sources et les capteurs permet de sonder l'échantillon à différentes profondeurs et, en utilisant des sources présentant un rayonnement proche d'une bande d'absorption de l'eau, permet de tracer un profil de la concentration en eau en fonction de la profondeur.

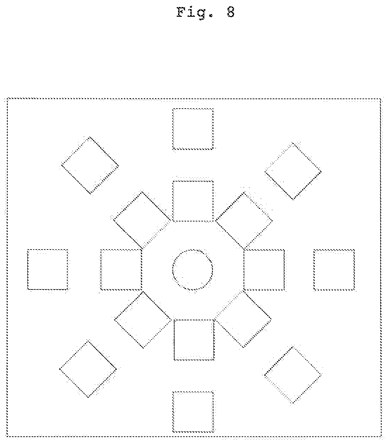

Dans une troisième variante illustrée par la figure 8, l'équipement comprend plusieurs sources (respectivement plusieurs capteurs) disposées de manière concentriques vis-à-vis du capteur (respectivement de la source). Cette réalisation permet d'homogénéiser spatialement les mesures et de mesurer le coefficient de diffusion réduit de l'échantillon selon plusieurs directions. Dans le cas de la peau humaine, l'évolution angulaire de ce coefficient peut être reliée à des caractéristiques liées au vieillissement de la peau.

Ces équipements peuvent présenter différentes caractéristiques et spécificités :

- Utilisation d'une ou plusieurs autres longueurs d'ondes pour mesurer la concentration en eau grâce à d'autres bandes d'absorption.

- Utilisation d'une ou plusieurs autres longueurs d'ondes pour mesurer le coefficient de diffusion réduit à différentes longueurs d'ondes.

- Capteur additionnel d'humidité et de température pour corréler les résultats de mesure de l'hydratation à l'humidité et à la température ambiante.

- Capteur additionnel des pertes insensibles en eau pour mesurer la dynamique des pertes en eau à travers l'épiderme et la couche cornée pour la peau.

- Capteur additionnel de couleur visibles.

- Positionnement des sources et des capteurs selon différentes hauteurs pour limiter l'illumination directe du ou des capteurs par les sources les plus proches.

- Positionnement de matériaux absorbant le rayonnement émis par les sources pour limiter l'illumination directe du ou des capteurs par les sources les plus proches.

- Utilisation la technique du temps de vol pour mesurer le coefficient de diffusion de l'échantillon.

- Travail à l'angle de Brewster en illuminant l'échantillon avec un rayonnement transverse magnétique ou en ne détectant que cette polarisation pour limiter l'influence des réflexions spéculaires sur l'échantillon.

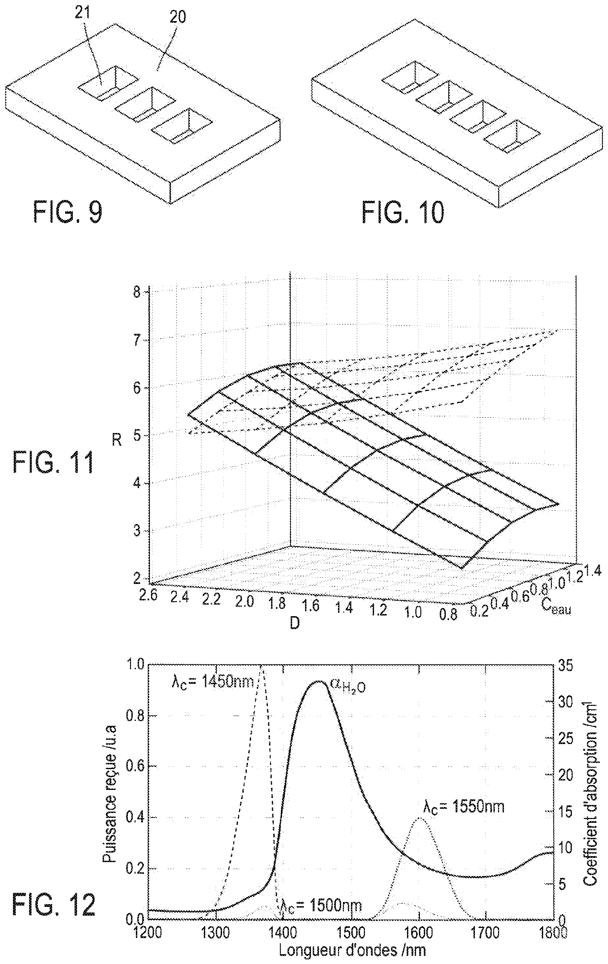

On décrit dans ce qui suit en référence aux figures 9 à 18, des modes de réalisation préférés de l'invention.

Le procédé selon l'invention s'attache à réaliser une mesure du coefficient de diffusion optique et de la concentration en eau d'un échantillon de peau à partir de signaux lumineux recueillis suite à une interaction entre une lumière incidente et une zone de la peau de relativement faible profondeur (d'une profondeur inférieure à une profondeur maximale inférieure à 2 millimètres , de préférence inférieure à 500 microns), signaux qui sont convertis en un couple « coefficient d'absorption/coefficient de diffusion » au moyen de fonctions préétablies à partir d'échantillons référents de peau reconstituée (dits échantillons fantômes) de constitution optimisée ou à partir de modèles numériques résolvant les équations de transfert radiatif physiques régissant l'interaction entre l'échantillon et la lumière émise par l'invention et calculant la puissance reçue.

En se limitant à une faible profondeur de peau, on limite l'interaction de la lumière traversant la zone peu profonde de la peau, avec des éléments (réseau veineux, de tissus musculaires ou osseux) perturbant autant sinon plus que les constituants de la peau, la progression de la lumière incidente, et parasitant ainsi la mesure de la concentration en eau.

Dans le cas où la profondeur maximale est d'environ 500 microns, la zone sondée est alors principalement le derme et les mesures réalisées peuvent alors être reliées au collagène présent dans le derme et la concentration en eau du derme.

Dans le cas où la profondeur maximale est d'environ 100 microns, la zone sondée est alors principalement l'épiderme et les mesures réalisées peuvent alors être reliées à la kératine présente dans l'épiderme et la concentration en eau de l'épiderme.

Un choix particulier de types et de nombre de sources de lumière incidente de longueurs d'onde spécifiques et de détecteurs de la lumière collectée après interaction avec la zone étudiée de l'échantillon de peau, permet de limiter à des signaux d'intensité lumineuse, les données nécessaires à la détermination du coefficient d'absorption et du coefficient de diffusion, alors que les procédés de l'état de la technique utilisent des spectres complets, et à des fonctions analytiques (définissant des surfaces tridimensionnelles ou des isocontours), les données résultant des échantillons fantômes ou des simulations numériques, quand les procédés de l'état de la technique nécessitent des matrices ou des bibliothèques de plusieurs mega, giga ou teraoctets pour répertorier les données issues de leurs échantillons fantômes.

Plus précisément, le principe de fonctionnement du procédé selon l'invention est le suivant :

On détecte un premier signal S1 en illuminant la peau par une première source lumineuse émettant à une longueur d'onde connue comme une longueur d'onde d'absorption de l'eau (LED par exemple) et en recueillant la lumière issue de la peau par un premier détecteur (photodiode par exemple). Ce premier signal équivaudrait à un sommet d'un pic d'absorption de l'eau à la longueur d'onde d'absorption choisie, sur un spectre d'absorption de l'échantillon acquis en fonction des longueurs d'onde.

On détecte un deuxième signal S2 qui équivaudrait au pied du pic d'absorption de l'eau précité, en illuminant la peau par une deuxième source lumineuse émettant à une longueur d'onde connue comme une longueur d'onde d'absence d'absorption de l'eau (LED par exemple) et en recueillant la lumière issue de la peau par le même détecteur (photodiode par exemple). Cette source lumineuse émettant à une longueur d'onde d'absence d'absorption de l'eau définit avec le détecteur, un premier couple d'analyse de la diffusion de l'échantillon définissant une première distance de séparation détecteur/source.

Ces deux premiers signaux simples de mesure, et de taille de stockage particulièrement faible, permettent d'obtenir une information qui équivaudrait à la hauteur d'un pic d'absorption de l'eau sur un spectre. Ils peuvent être exploités pour obtenir le coefficient d'absorption de l'eau, et le coefficient de diffusion de la lumière, conformément aux figures 4 et 5 et à la description qui en est faite précédemment. Cependant, il est préférable de procéder à l'acquisition d'au moins un troisième signal car la quantité d'eau dans la peau va modifier le saut d'indice optique avec les diffuseurs et donc modifier le coefficient de diffusion de la lumière.

De préférence, on détecte donc un troisième signal S3 à partir d'un deuxième couple « source lumineuse émettant à une longueur d'onde d'absence d'absorption de l'eau / détecteur » définissant une deuxième distance de séparation, différente de la première.

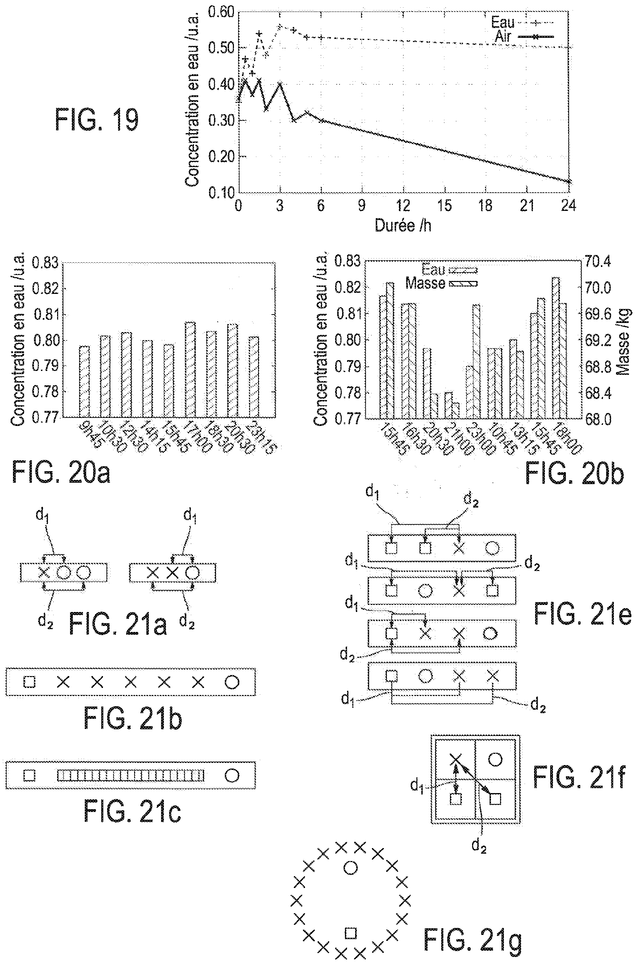

Les couples sources/capteurs peuvent être définis de diverses manières à partir de divers agencements de capteurs/sources lumineuses d'équipements selon différentes configurations de l'invention dont certains exemples sont représentés sur les figures 21a) à 21g) et seront explicités ultérieurement.

Réalisation de modèles théoriques (non-couverte par les revendications)

En résolvant l'équation de transfert radiatif pour un agencement de sources et de capteurs grâce à des algorithmes Monte Carlo par exemple pour des simulations dans lesquelles on fait varier la concentration en eau et le coefficient de diffusion, on calcule les trois signaux reçus (S1 : signal résultant d'une illumination de l'échantillon fantôme par une source émettant à une longueur d'onde d'absorption de l'eau, S2 : signal résultant d'une illumination de l'échantillon fantôme par une source émettant à une longueur d'onde d'absence d'absorption de l'eau définissant avec le capteur, une première distance, S3 : signal résultant d'une illumination de l'échantillon fantôme par une source émettant à une longueur d'onde d'absence d'absorption de l'eau définissant avec le capteur, une deuxième distance). Des équations pour chaque signal peuvent alors être recherchées pour obtenir la bonne adéquation entre ces calculs et les conditions de concentration en eau et de coefficient de diffusion optiques. Ces équations peuvent faire appel à titre d'exemples non limitatifs à des fonctions polynomiales, exponentielles, logarithmiques ou hyperboliques.

Réalisation d'abaques (selon l'invention)

A partir d'échantillons de concentration en eau connue et de coefficient de diffusion connu, nommés « échantillons fantômes », on réalise pour un agencement donné de sources et de capteurs d'un équipement selon l'invention, des abaques pour les trois signaux mesurés (S1 : signal résultant d'une illumination de l'échantillon fantôme par une source émettant à une longueur d'onde d'absorption de l'eau, S2 : signal résultant d'une illumination de l'échantillon fantôme par une source émettant à une longueur d'onde d'absence d'absorption de l'eau définissant avec le capteur, une première distance, S3 : signal résultant d'une illumination de l'échantillon fantôme par une source émettant à une longueur d'onde d'absence d'absorption de l'eau définissant avec le capteur, une deuxième distance).

Des abaques sont représentés sur les figures 14 a) à c) telles qu'obtenues pour le premier exemple d'agencement de capteurs/sources de la figure 21e).

On constate avec une représentation tridimensionnelle des mesures obtenues, que pour chacun des trois signaux S1, S2, S3 il est possible à partir des trios obtenus pour les différents échantillons fantômes (Signal S1, S2 ou S3 mesuré, coefficient d'absorption, coefficient de diffusion) de définir des fonctions relativement simples en ce sens les signaux S1, S2, S3 obtenus pour l'ensemble des échantillons fantômes, peuvent chacun être représentés par une surface continue dont l'évolution ou la courbure peut être interprétée scientifiquement:

-   évolution S1 : pour des échantillons fantômes présentant un faible coefficient d'absorption et un faible coefficient de diffusion, le signal S1 est relativement constant car l'absorption et la diffusion se compensent mutuellement, puis le signal S1 chute pour des coefficients d'absorption plus élevés, l'absorption l'emportant sur la diffusion,

-   évolution S2 : pour les faibles concentrations en eau, le signal de lumière diffusée augmente avec le coefficient de diffusion des échantillons fantômes, puis diminue lorsque le coefficient d'absorption des échantillons augmente puisque la concentration des diffuseurs diminue alors

-   évolution S3 : à coefficient d'absorption constant, le signal diminue lorsque le coefficient de diffusion augmente, la lumière incidente étant diffusée dans la masse de l'échantillon d'autant plus que le nombre de diffuseur est important ; à coefficient de diffusion constant, le signal diminue lorsque la concentration en eau augmente.

Ce résultat est remarquable car il permet de définir des fonctions analytiques simples pour chaque signal Si qui sont obtenues par mesures directes sur les échantillons fantômes. Et pour un échantillon de peau de coefficient d'absorption et de diffusion inconnu, il suffira de mesurer avec le même équipement que celui ayant permis la définition de ces abaques, les trois signaux S1, S2, S3, de positionner ces valeurs sur les abaques et s'il existe une duo unique (coefficient d'absorption, coefficient de diffusion) satisfaisant à la fois les trois fonctions analytiques définies par les abaques, il sera possible d'assimiler ce duo unique à la concentration en eau et au coefficient de diffusion de l'échantillon inconnu, par exemple par une technique de minimisation des moindres carrés.

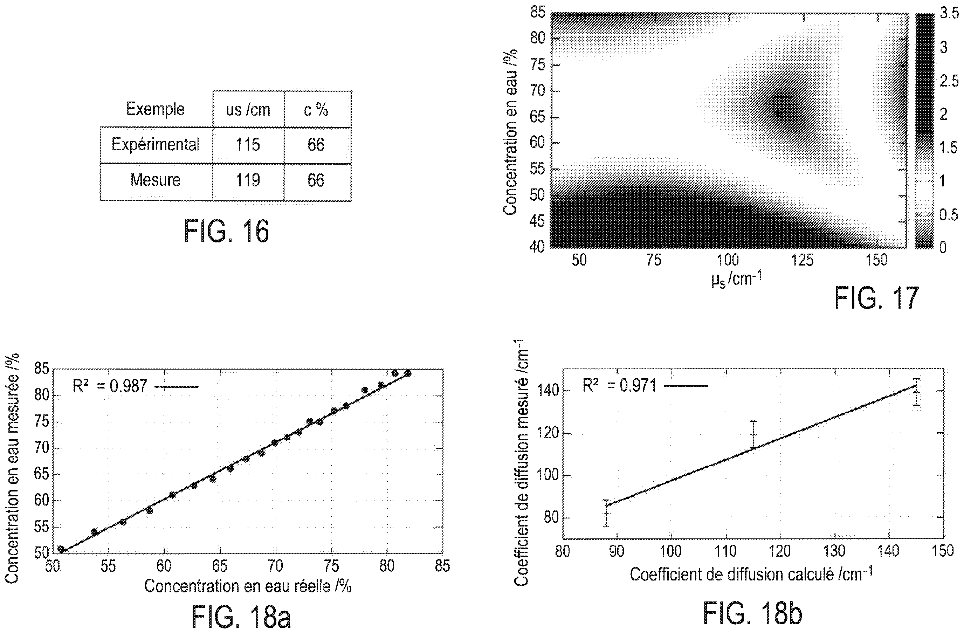

La figure 18a) montre la bonne corrélation obtenue pour des échantillons tests de coefficients de diffusion fixés, entre la concentration en eau mesurée et leur concentration en eau connue.

La figure 18b) montre la bonne corrélation obtenue pour des échantillons tests de concentration en eau donnée, entre le coefficient de diffusion mesuré et leur coefficient de diffusion connu.

Les abaques préparés peuvent donc permettre la détermination de la concentration en eau et du coefficient de diffusion d'échantillons de peau inconnus, tout en requérant pour leur stockage en mémoire un minimum d'espace, comparé aux matrices ou bibliothèques de données des procédés connus.

Dispositif à trois signaux

Ce type de dispositif permet l'obtention de l'estimation de la concentration en eau d'un échantillon inconnu, et celle de son coefficient de diffusion de la lumière à partir de laquelle il est possible d'obtenir une information sur la qualité/quantité de son collagène ou de sa kératine.

Configuration générale

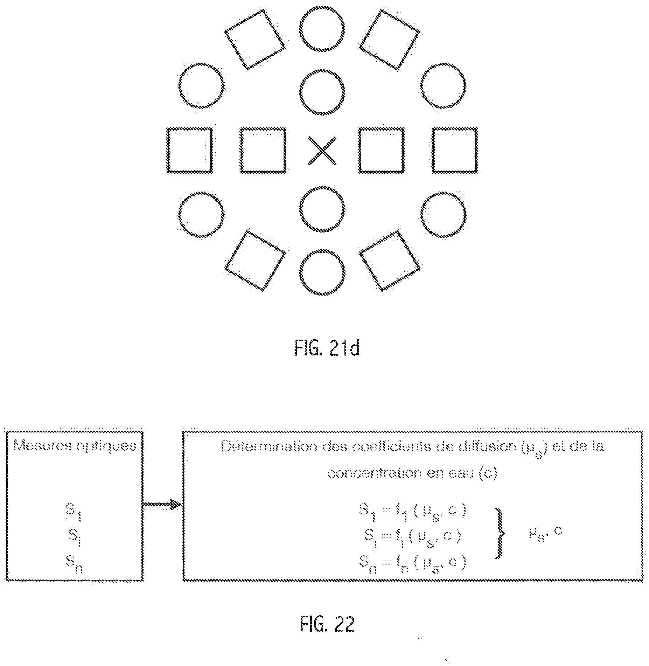

Conformément aux figure 21 e) et f), un dispositif de ce type est pourvu au minimum :

- d'un premier couple de composants formé d'une source de lumière (LED) émettant un spectre de lumière proche d'un pic d'absorption de l'eau pour lequel le coefficient effectif d'absorption est élevé et d'un capteur (photodiode), définissant une première distance de séparation entre le capteur et la source

- d'un deuxième couple de composants formé d'une source de lumière (LED) émettant un spectre de lumière centré autour d'une longueur d'absence ou de bien moindre absorption de l'eau (par ex. 1300 nm voir spectre de l'eau de la figure 12) et d'un capteur (photodiode), définissant une première distance de séparation entre le capteur et la source

- d'un troisième couple de composants formé d'une seconde source de lumière (LED) émettant un spectre de lumière centré autour d'une longueur d'absence ou de bien moindre absorption de l'eau (par exe. 1300 nm voir spectre de l'eau de la figure 12) et d'un capteur (photodiode), définissant une deuxième distance de séparation.

Le choix des longueurs d'onde des sources de lumière, qui sont généralement des LEDs, est guidé par les considérations suivantes :
La concentration de l'eau dans la peau se situe vers 65%. L'eau présente un spectre représenté sur la figure 12, présentant un pic d'absorption centré autour de 1450 nm et présentant une absorption moyenne. C'est ce pic d'absorption qui sera préférentiellement exploité pour les besoins de l'invention.

A cet effet, sachant que les LED ont une largeur spectrale non négligeable dans cette gamme spectrale, lors du choix de la LED comme première ou deuxième source de lumière permettant de déterminer la concentration en eau, il convient de choisir une LED dont le pic d'émission est centré sur une longueur d'onde supérieur au pic de l'eau choisi (1450 nm). Ceci permet d'éviter un débordement trop marqué vers 1350 nm obtenu avec une source dont le pic d'émission serait centré à une longueur d'onde inférieure à 1450 nm (courbe repérée par λc = 1450 nm sur la figure 12). Des sources dont les pics d'émission sont centrés vers 1500 nm ou 1550 nm permettent donc de gagner en dynamique et seront préférées.

Pour la troisième source permettant la caractérisation de la diffusion, on choisit une longueur d'onde d'émission en dehors du pic d'absorption de l'eau, et bien inférieur à celui-ci, par exemple 1300 nm.

### Différents agencements possibles

Les figures 21 e) et f) précitées illustrent différents agencements répondant à ces exigences :
Les deux premiers dispositifs de la figure 21 e) et le dispositif 21 f) constitués d'un capteur (représenté par une croix), de deux sources centrées autour d'une longueur d'onde d'absence d'absorption de l'eau (représentées chacune par un carré) et définissant avec le capteur respectivement deux distances de mesure (d1, d2), et une source dont le spectre est proche d'un pic d'absorption de l'eau, positionnée au plus proche du capteur, préférentiellement à la distance d1 (et représentée par un cercle).

Les deux derniers dispositifs de la figure 21 e) constitués de deux capteurs (représentés par une croix), et de deux sources centrées respectivement autour d'une longueur d'onde d'absence d'absorption de l'eau (représentée par un carré), et une source dont le spectre est proche d'un pic d'absorption de l'eau, positionnée au plus proche du capteur. Les deux capteurs définissent avec la source centrée autour d'une longueur d'onde d'absence d'absorption de l'eau, deux distances de mesure (d1, d2).

Les agencements perfectionnés des figures 8, 21 b), 21c) ou 21d permettent d'obtenir plusieurs signaux représentatifs de l'absorption d'eau intervenant sur différentes couches de différentes profondeurs de l'échantillon étudié grâce aux multiples couples définis entre la source émettant autour d'une longueur d'absorption de l'eau et les différents capteurs, et également plusieurs signaux représentatifs de la diffusion des particules présentes sur différentes couches de différentes profondeurs de l'échantillon étudié grâce aux multiples couples définis entre la source émettant autour d'une longueur d'absence d'absorption de l'eau et les différents capteurs.

La barrette linéaire de photodiode de la figure 21c) permet d'obtenir des informations pratiquement continues sur la concentration en eau et la qualité du collagène et/ou de la kératine sur toute la profondeur de la zone étudiée.

Les agencements proposés par les dispositifs de la figure 21g) dans lesquels les sources ou les capteurs (photodiodes) sont répartis en anneau autour respectivement d'un capteur excentré ou d'une source excentrée (ou deux sources distinctes conformément à l'illustration), de façon à définir différentes distances d'analyse di et des mesures de confirmation. On pourrait prévoir pour plus d'informations sur une zone, une multitude d'anneaux concentriques comme l'illustre les figure 8) et 21d).

### Exploitation de l'information portant sur le coefficient de diffusion

L'information obtenue suite à la mise en oeuvre du procédé selon l'invention sur le coefficient de diffusion peut être exploitée pour obtenir des informations qualitative sur le collagène (organisé en fibres longues relativement parallèles ou désorganisé en vermicelles anarchiques) ou sur la kératine en fonction de la profondeur de la mesure. Les différents agencements de dispositifs permettant des mesures de coefficients de diffusion d'un échantillon selon plusieurs directions (figure 8, 21d), 21g)) peuvent être exploitées pour obtenir des informations sur l'évolution qualitative et quantitative du collagène dans les différentes directions de l'échantillon étudié.

Le moyen d'affichage pourra présenter une information relative au collagène ou à la kératine en plus de celle relative à la concentration en eau.

Masque

Le dispositif comprend avantageusement un masque tel représenté sur les figures 9 et 10 pour les agencements à trois éléments de la figure 21a) et pour les agencement à quatre éléments de la figure 21b). Ce masque est destiné à s'interposer physiquement entre une source et un capteur adjacent pour prévenir son illumination directe, et forme en outre autour de chaque source, un tunnel de concentration en une zone précise de la lumière émise.

Ce masque 20, est formé par un pavé massique, réalisé en matériau opaque aux longueurs d'onde utilisées, qui comprend des fenêtres traversantes 21 dans l'épaisseur du pavé, et ouvertes sur ses grandes faces. Il peut être réalisé en matière plastique ou en matière métallique. Les fenêtres et/ou les poutres définies entre celles ci, d'une largeur de 50 microns à 1 mm peuvent être obtenues par mécanique de précision via injection plastique, découpe laser ou électroérosion à fil. Ce masque est appliqué sur le dispositif pour lequel il a été conçu, de façon à faire coïncider ses fenêtres avec les sources et détecteurs présents sur le dispositif, puis une résine en matériau biocompatible est appliquée sur le masque pour combler les fenêtres, est durcie et polie pour obtenir les dimensions et le fini désirés.

Cette résine pourra être revêtue d'une couche de gel à base de silicone ou d'huile protégée par une pellicule amovible à ôter pour effectuer une mesure sur la peau d'un individu.

Préparation des échantillons fantômes optimisés

La possibilité de définir des fonctions analytiques relativement simples et de tailles de stockage particulièrement réduites, à partir de ces abaques, peut être liée au choix du nombre de signaux mesurés, à la faible profondeur d'échantillon de peau analysée, et également à la nature même des échantillons fantômes préparés à partir desquels sont réalisées les abaques et indirectement, les mesures.

On décrit dans ce qui suit les problématiques rencontrées dans des échantillons fantômes connus et la solution proposée par l'invention.

L'absence de connaissance sur les constituants d'un matériau biologique que l'on souhaite étudier, et leurs concentrations dans celui-ci conduit très souvent à la réalisation d'échantillons de référence (ou échantillons fantômes) pour réaliser des preuves de concept ou calibrer des dispositifs.

Ces échantillons fantômes, réalisés artificiellement, ont pour but de se rapprocher structurellement du matériau réel à étudier, en comptant cependant des constituants de nature et de concentration connues permettant la qualification ou la calibration de nouveaux dispositifs ou de nouvelles méthodes de mesure.

Dans le cas de la caractérisation d'un matériau biologique par sa concentration en diffuseurs et en absorbeurs de la lumière, les paramètres suivants doivent être pris en compte :

- l'indice de réfraction optique des diffuseurs,

- la forme des diffuseurs ou leur distribution en forme,

- la taille des diffuseurs ou leur distribution en taille,

- le poids spécifique des diffuseurs,

- la concentration volumique des diffuseurs,

- l'indice de réfraction optique du milieu qui entoure les diffuseurs (le solvant),

- le poids spécifique du solvant,

- la concentration volumique du ou des absorbants lumineux qui sont dilués dans le solvant.

Spécifiquement, dans le cas de l'étude de la concentration en eau et de la diffusion optique du derme, produite principalement par le collagène, on réalise usuellement des échantillons fantômes constitués :

- de particules diffusantes sous forme de poudre (dioxyde de titane ou dioxyde de silicium

- d'un solvant formé d'un mélange eau-diluant miscible avec l'eau et qui présente une absorption très faible aux longueurs d'onde de travail, comme par exemple le diméthylsulfoxyde ou le deutérium.

Les échantillons fantômes réalisés pour simuler la peau, négligent très souvent l'influence de l'anisotropie de la diffusion en choisissant des particules diffusantes qui reproduisent mal sa valeur (elle vaut environ 0.9 dans le cas de la peau).

Cependant, ces échantillons connus ne reproduisent pas de façon satisfaisante l'anisotropie de la diffusion en raison des particules diffusantes choisies, qui en reproduisent mal la valeur (environ 0,9 dans le cas de la peau).

Ils ne reproduisent pas non plus correctement la peau en terme de diffusion optique car ce phénomène repose dans la peau réelle, sur la variation d'indice optique entre les particules diffusante et l'eau, et dans l'échantillon fantôme, non seulement comme pour la peau sur la variation d'indice optique entre les particules et l'eau ajoutée, mais également et de façon parasitaire, sur la variation d'indice optique entre les particules et le solvant diluant utilisé pour simuler le milieu biologique de la peau.

Ainsi, les mesures effectuées ne reproduisent pas les conditions réelles et induisent, au mieux, un décalage par rapport aux mesures in-vivo, au pire, une inadéquation complète entre les mesures faites sur les échantillons fantômes et les mesures faites dans les conditions réelles. Toute calibration de dispositifs est ainsi rendue caduque.

L'objectif de l'invention a été de pallier ces inconvénients pour obtenir des échantillons fantômes qui reproduisent le plus fidèlement possible, notamment en matière d'anisotropie et de variation d'indice avec l'absorbant, le comportement optique du matériau à analyser. Plus particulièrement dans le cas de la peau, il s'agit de reproduire le comportement optique du derme ou de l'épiderme.

Cet objectif est atteint en :

- Utilisant des particules diffusantes dont la forme, la taille (ou leurs distributions) et le saut d'indice avec le solvant permettent de reproduire l'anisotropie du milieu à étudier.

- Ajoutant un constituant supplémentaire dans le solvant pour que la variation d'indice entre les particules et le solvant provienne uniquement de l'ajout de l'absorbant (l'eau) dans le solvant.

Les étapes du procédé de fabrication d'échantillons tests de l'invention comprennent :

a) mélange entre un diluant et des particules diffusantes,

b) ajout d'un constituant intermédiaire pour annuler la diffusion des particules dans le diluant et qui va modifier l'indice optique du diluant pour le faire égaler celui des particules et rendre le mélange transparent aux longueurs d'onde de travail(dans le sens non diffusant)

c) ajout de l'absorbant

Une fois le quatuor diluant/particules/absorbant/composant intermédiaire trouvé, on réalise différents échantillons fantômes avec des quantités d'absorbant et de particules différentes et connues.

*Détermination des constituants de l'échantillon de référence*

a) Choix de l'absorbant : son spectre d'absorption, son indice de réfraction et son poids spécifiques doivent être les plus proches possible de ceux de l'absorbant à étudier. Il est parfois possible d'utiliser directement l'absorbant présent dans le matériau à analyser, en l'occurrence, l'eau ou l'eau désionisée pour l'exemple de l'étude de la concentration en eau de la peau.

b) Choix du diluant : son indice de réfraction et son poids spécifique doivent être les plus proches possible de ceux de l'absorbant tandis que son absorption optique dans la gamme de longueurs d'ondes étudiée (longueur d'onde d'absorption de l'eau et longueur d'onde d'absence d'absorption de l'eau) doit être négligeable devant celle de l'absorbant. Il est parfois possible d'utiliser les isotopes de l'absorbant.

c) Choix des particules diffusantes : leur indice de réfraction et leur poids spécifique doivent être les plus proches possible de celui des diffuseurs dans les conditions réelles(le collagène ou la kératine dans le cas de l'étude de la peau). De plus, leur taille (ou leur distribution en taille) doit permettre d'obtenir une anisotropie proche de celle des conditions réelles.

d) Choix du constituant intermédiaire : lors de son ajout dans le mélange diluant/absorbant, l'ajout d'un constituant intermédiaire doit permettre d'égaliser l'indice de réfraction du diluant et l'indice de réfraction des particules diffusantes avant l'ajout de l'absorbant.

On peut noter que les étapes a), b) et c) peuvent être interchangées.

L'objectif est d'obtenir des concentrations en absorbant, des coefficients de diffusion et une anisotropie qui soient les plus proches possibles de conditions réelles :

- la concentration en absorbant est directement contrôlée par la quantité d'absorbant ajoutée au solvant,
- le coefficient de diffusion et l'anisotropie sont contrôlés via des modèles de diffusion optique qui permettent de les calculer : diffusion de Mie, diffusion de Rayleigh, cas des particules sphériques... ; et de déterminer pour un coefficient de diffusion et une anisotropie à atteindre, des formes et des tailles de particules données
- en plus de devoir être transparent aux longueurs d'onde de travail, le choix du constituant intermédiaire est principalement guidé par le choix de son indice de réfraction, qui est donné par la formule suivante :

$$n_p = c_i * n_i + c_d * n_d$$

$$\text{soit } n_i = (np - c_d * n_d) / c_i$$

où $n_p$, $n_i$ et $n_d$ sont respectivement les indices de réfraction des particules diffusantes, du constituant intermédiaire et du diluant et $c_i$ et $c_d$ sont respectivement les concentrations massiques du constituant intermédiaire et du diluant dans le mélange.

*Exemple : cas du derme*

Ce principe de rendre transparent aux longueurs d'ondes de travail le mélange du diluant et des particules est transposable à différents types de matériaux à simuler par échantillons fantômes pour étudier différents types de composants absorbant la lumière.

Dans le cas de la simulation du derme par échantillon fantôme, pour évaluer la teneur en eau et l'état du collagène d'échantillons inconnus, ce derme peut selon le procédé de préparation des échantillons fantômes selon l'invention, être modélisé par un ensemble de particules diffusantes (le collagène) qui baigne dans un milieu absorbant (l'eau).

Pour reproduire les caractéristiques optique du derme, on peut par exemple utiliser un mélange comportant :

- de l'eau (l'absorbant),

- de l'eau lourde (le diluant)

- des particules de dioxyde de titane ou de silicium sphériques dont le diamètre est centré vers 1,5 $\mu$m.

- de l'hydrazine ou de la pyridine (le constituant intermédiaire)

Cette combinaison permet d'obtenir facilement des coefficients de diffusion adaptés (50 /cm -> 150 /cm) pour des concentrations en eau classiques dans le derme (50% -> 80%) et avec une anisotropie de 0,89 (dans le cadre de la diffusion de Mie en présence de particules sphériques).

D'autres combinaisons sont également envisageables, notamment concernant le choix du constituant intermédiaire.

*Formation d'échantillons fantômes solides*

Une réalisation avantageuse consiste à rendre le mélange solide. Pour cela, des étapes de solidification avec des agents de durcissement (de type silicone par exemple), d'élastomères (de type silicone par exemple) ou de produits gélifiants (de type gélatine ou agar agar par exemple) avec éventuellement des étapes de recuit et de dégazement sont ajoutées.

Preuves de concept

Des mesures ex-vivo (figure 19) ont été réalisées sur des prélèvements de peaux de cochon laissées sécher à l'air libre et des peaux en contact avec du liquide physiologique à l'aide du dispositif de la figure 21e). Les concentrations en eau des échantillons laissés à l'air libre, telles que fournies par le procédé selon l'invention, diminuent en fonction du temps d'exposition à l'air montrant leur séchage progressif, alors que celles de l'échantillon en contact avec du liquide physiologique sont relativement constantes en fonction du temps, montrant la constance de leur teneur en eau.

Des mesures in vivo effectuées à l'aide du dispositif de la figure 21e) sur une personne volontaire (derme du mollet) lors d'une journée sans activité sportive particulière (figure 20a) et lors d'une journée interrompue par une séance de sport à une heure précise (16h30) sans que le volontaire ne boive ensuite jusqu'à 21 h30 puis dîne, par le procédé selon l'invention montrent la constance de la concentration en eau mesurée (figure 20a), et la chute de la concentration en eau à partir de la séance de sport puis sa remontée progressive (figure 20b), évolution corroborée par les mesures du poids du volontaire qui indiquent une perte d'eau d'environ 3% et une remontée de poids à compter du dîner.

**Revendications**

1. Procédé de mesure de la concentration de l'eau dans un matériau diffusant la lumière comportant les étapes suivantes :

   - émission par N sources lumineuses (2, 3) $SL_i$- de faisceaux de longueurs d'onde Lambda; sur le matériau
   - acquisition par M capteurs (4) $P_j$ sensibles dans une partie au moins desdites longueurs d'onde Lambda; de signaux $S_j$ émis par le matériau
   - avec M+N$\geq$3 le procédé comportant en outre les étapes de :
   - calcul d'une première information $I_{dif}$ représentative de la diffusion, et une deuxième information $I_{abs}$ représentative de l'absorption, en fonction desdits signaux $S_j$ et d'une information numérique représentative du matériau diffusant d'une part et desdites sources $SL_i$ et desdits capteurs $P_j$ d'autre part,
   - calcul de ladite concentration d'eau en utilisant la deuxième information Iabs,

   le procédé étant **caractérisé en ce que** l'information numérique est fournie par une ou des fonctions dont la détermination est réalisée par une étape de calibration par acquisition des signaux Sj pour des échantillons chacun composés d'au moins un absorbant, un diluant, un diffusant utilisant des particules diffusantes dont la forme, la taille ou leur distribution en taille permettent de reproduire l'anisotropie du milieu à étudier et un constituant intermédiaire choisi de telle sorte à égaliser l'indice de réfraction du diluant et l'indice de réfraction des particules diffusantes avant l'ajout de l'absorbant dans l'échantillon.

**2.** Procédé de mesure de la concentration de l'eau selon la revendication 1 **caractérisé en ce que** la longueur d'onde Lambda; est choisie proche de 1450 ou 1940, ou 970 ou 1190 nanomètres, pour les sources lumineuses et capteurs délivrant un signal représentatif de la diffusion dans une longueur d'onde correspondant à un pic absorption de l'eau.

**3.** Procédé de mesure de la concentration de l'eau selon la revendication 1 **caractérisé en ce que** la longueur d'onde Lambda; est choisie proche de 1050 ou 1070 ou 1100 ou 1300 nanomètres, pour les sources lumineuses et capteurs délivrant le signal représentatif de la diffusion dans une longueur d'onde différent d'un pic absorption de l'eau.

**4.** Equipement de mesure de la concentration de l'eau dans un matériau diffusant la lumière comprenant N sources lumineuses (2, 3) $SL_i$ émettant dans des longueurs d'onde Lambda; et M capteurs (4) $P_j$ sensibles dans une partie au moins desdites longueurs d'onde Lambda;, avec $N+M \geq 3$, un circuit électronique comprenant pour chacun desdits capteurs $P_j$ un moyen de traitement du signal pour délivrer un signal $S_j$, un traitement et un calculateur commandant le traitement délivrant, en fonction desdits signaux $S_j$ et d'une information numérique représentative du matériau diffusant et de la géométrie desdites sources $SL_i$ et desdits capteurs $P_j$, une première information $I_{dif}$ représentative de la diffusion, une deuxième information $I_{abs}$ représentative de l'absorption et délivrant ladite concentration en eau en fonction de la deuxième information $I_{abs}$ **caractérisé en ce que** l'équipement comprend un ou plusieurs échantillons de calibration et **en ce que** l'information numérique est fournie par une ou des fonctions dont la détermination est réalisée par une étape de calibration par acquisition des signaux Sj pour les échantillons de calibration chacun composés d'au moins un absorbant, un diluant, un diffusant utilisant des particules diffusantes dont la forme, la taille ou leur distribution en taille permettent de reproduire l'anisotropie du milieu à étudier et un constituant intermédiaire choisi de telle sorte à égaliser l'indice de réfraction du diluant et l'indice de réfraction des particules diffusantes avant l'ajout de l'absorbant dans l'échantillon.

**5.** Equipement selon la revendication précédente **caractérisé en ce que** lesdites N sources lumineuses $SL_i$ et lesdits M capteurs $P_j$ sont logées dans un support s'ouvrant par une fenêtre de contact avec le matériau à caractériser, ledit support comportant un écran empêchant la transmission directe et/ou réfléchie, sans pénétration préalable dans ledit matériau diffusant, de la lumière entre une source lumineuse et un capteur, et **en ce que** la fenêtre du support présente un traitement anti-reflet.

**6.** Equipement selon les revendications 4 ou 5, **caractérisé en ce que** lesdites N sources lumineuses $SL_i$ sont polarisées et en ce la direction du faisceau émis forme un angle de Brewster avec l'axe optique desdits M capteurs $P_j$.

**7.** Equipement selon l'une des revendications 4 à 6, **caractérisé en ce qu'**il comprend des moyens de radiocommunication pour transmettre périodiquement le résultat de la mesure à un équipement distant.

**8.** Equipement selon l'une des revendications 4 à 7, **caractérisé en ce que** les sources lumineuses, les capteurs et le circuit électronique constituent un patch présentant des moyens de liaison réversible sur un patient.

**9.** Equipement selon l'une des revendications 4 à 8, **caractérisé en ce qu'**il comprend au moins deux couples source lumineuse/ capteur sensible, un premier couple source lumineuse/capteur sensible définissant entre le capteur et la source une première distance de séparation, et un deuxième couple source lumineuse/capteur sensible définissant une deuxième distance de séparation.

**10.** Equipement selon la revendication précédente, **caractérisé en ce que** les sources des deux couples émettent toutes deux avec un spectre centré autour d'une longueur d'onde proche de ou supérieure à une longueur d'onde d'absorption de l'eau ou **en ce que** l'une des deux sources émet avec un spectre centré autour d'une longueur d'onde proche d'un pic d'absorption de l'eau et l'autre source, avec un spectre centré autour d'une longueur d'onde d'absence d'absorption de l'eau, et **en ce que** les sources des deux couples émettent toutes deux avec un spectre centré autour d'une longueur d'onde d'absorption de 1450 nm, 1500 nm ou 1550 nm.

**11.** Equipement selon la revendication 9, **caractérisé en ce que** les sources des deux couples émettent toutes deux dans une longueur d'onde d'absence d'absorption de l'eau, **en ce que** les sources des deux couples émettent toutes deux avec un spectre centré autour de 1300 nm.

**12.** Equipement selon l'une des revendications 9 à 11, comprenant un troisième couple source lumineuse/capteur, la source lumineuse émettant dans une longueur d'onde d'absorption de l'eau, qui est disposée au plus proche du capteur, comprenant un capteur et trois sources, les trois sources appartenant respectivement à trois couples source/capteur distincts, ou comprenant deux capteurs et deux sources, l'une des deux sources émettant dans une longueur d'onde proche d'un pic d'absorption de l'eau,

et l'autre source dans une longueur d'onde d'absence d'absorption de l'eau.

13. Equipement selon la revendication 10, comprenant entre les deux sources distinctes une série de capteurs distincts disposés en ligne ou une barrette linéaire de photodiodes.

14. Equipement selon l'une des revendications 9 à 13, comprenant un capteur central ou excentré et autour de ce capteur une succession de sources d'émission lumineuse centrées autour de longueurs d'ondes d'absorption et/ou d'absence d'absorption de la lumière.

15. Equipement selon l'une des revendication 9 à 14, comprenant au moins une source d'émission lumineuse centrale ou excentrée émettant avec un spectre de lumière centré autour d'une longueur d'ondes d'absorption ou d'absence d'absorption de la lumière et autour de cette source, une succession de capteurs.

16. Equipement selon l'une des revendications 4 à 15, comprenant un masque s'interposant physiquement entre une source et un capteur adjacent, le masque formant autour de chaque source un tunnel de concentration en une zone précise de la lumière émise.

17. Application du procédé et de l'équipement selon une quelconque des revendications 1 à 16, pour la caractérisation de la concentration d'eau dans une matrice alimentaire, dans un végétal, ou dans une matrice cellulosique, tel que papier, carton et bois.

18. Application du procédé et de l'équipement selon une quelconque des revendications 1 à 16, pour la caractérisation de l'hydratation de la peau et/ou pour la caractérisation de la qualité et/ou la quantité de collagène ou de la kératine de la peau.

19. Application du procédé et de l'équipement selon une quelconque des revendications 1 à 16, pour la caractérisation de l'hydratation d'une mèche de cheveux.

**Patentansprüche**

1. Verfahren zur Messung der Wasserkonzentration in einem lichtstreuenden Material, das die folgenden Schritte aufweist:

- Emittieren, durch N Lichtquellen (2, 3) $SL_i$, von Strahlen der Wellenlängen Lambdai auf das Material,
- Erfassen, durch M Sensoren (4) $P_j$, die in mindestens einem Teil der Wellenlängen Lambda i empfindlich sind, von Signalen $S_j$, die von dem Material emittiert werden,
- mit M+N≥3,

wobei das Verfahren ferner die Schritte aufweist:

- Berechnen einer ersten Information $I_{dif}$, die die Streuung darstellt, und einer zweiten Information $I_{abs}$, die die Absorption darstellt ist, in Abhängigkeit von den Signalen $S_j$ und einer digitalen Information, die das streuende Material einerseits und die Quellen $SL_i$ und die Sensoren $P_j$ andererseits darstellt,
- Berechnen der Wasserkonzentration unter Verwendung der zweiten Information $I_{abs}$,

wobei das Verfahren **dadurch gekennzeichnet ist, dass** die digitale Information durch eine oder mehrere Funktionen bereitgestellt wird, deren Bestimmung durch einen Kalibrierungsschritt durch Erfassen der Signale Sj für Proben erfolgt, die jeweils bestehen

aus mindestens einem Absorptionsmittel, einem Verdünnungsmittel, einem Streumittel, das Streupartikel verwendet, deren Form, Größe oder Größenverteilung die Anisotropie des zu untersuchenden Mediums nachbilden können, und einer

Zwischenkomponente, die so gewählt ist, dass der Brechungsindex des Verdünnungsmittels und der Brechungsindex der Streupartikel vor der Zugabe des Absorptionsmittels in die Probe ausgeglichen werden.

2. Verfahren zur Messung der Wasserkonzentration nach Anspruch 1, **dadurch gekennzeichnet, dass** die Wellenlänge Lambdai nahe 1.450 oder 1.940 oder 970 oder 1.190 Nanometer gewählt wird, damit die Lichtquellen und Sensoren ein Signal liefern, das die Streuung in einer Wellenlänge darstellt, die einem Absorptionspeak von Wasser entspricht.

3. Verfahren zur Messung der Wasserkonzentration nach Anspruch 1, **dadurch gekennzeichnet, dass** die Wellenlänge Lambdai nahe 1.050 oder 1.070 oder 1.100 oder 1.300 Nanometer gewählt wird, damit die Lichtquellen und Sensoren das Signal liefern, das die Streuung in einer Wellenlänge darstellt, die sich von einem Absorptionspeak von Wasser unterscheidet.

4. Vorrichtung zur Messung der Wasserkonzentration in einem lichtstreuenden Material, umfassend N Lichtquellen (2, 3) $SL_i$, die in Wellenlängen Lambdai emittieren, und M Sensoren (4) $P_j$, die in mindestens einem Teil der Wellenlängen Lambdai empfindlich sind, mit N+M$\geq$3,

eine elektronische Schaltung, umfassend für jeden

der Sensoren $P_j$ ein Signalverarbeitungsmittel, um ein Signal $S_j$ zu liefern, eine Verarbeitung und einen Rechner, der die Verarbeitung steuert, die in Abhängigkeit von den Signalen $S_j$ und einer digitalen Information, die das streuende Material und die Geometrie der Quellen SL und der Sensoren $P_j$ darstellt, eine erste Information $I_{dif}$, die die Streuung darstellt, und eine zweite Information $I_{abs}$ liefert, die die Absorption darstellt und die Wasserkonzentration in Abhängigkeit von der zweiten Information $I_{abs}$ liefert, **dadurch gekennzeichnet, dass** die Vorrichtung eine oder mehrere Kalibrierungsproben umfasst und dadurch, dass

die digitale Information durch eine oder mehrere Funktionen bereitgestellt wird, deren Bestimmung durch einen Kalibrierungsschritt durch Erfassen der Signale Sj für die Kalibrierungsproben erfolgt, die jeweils bestehen

aus mindestens einem Absorptionsmittel, einem Verdünnungsmittel, einem Streumittel, das Streupartikel verwendet, deren Form, Größe oder Größenverteilung die Anisotropie des zu untersuchenden Mediums nachbilden können, und einer

Zwischenkomponente, die so gewählt ist, dass der Brechungsindex des Verdünnungsmittels und der Brechungsindex der Streupartikel vor der Zugabe des Absorptionsmittels in die Probe ausgeglichen werden.

5. Vorrichtung nach dem vorstehenden Anspruch, **dadurch gekennzeichnet, dass** die N Lichtquellen $SL_i$ und die M Sensoren $P_j$ in einem Träger untergebracht sind, der sich durch ein Fenster für den Kontakt mit dem zu charakterisierenden Material öffnet, wobei der Träger eine Abschirmung aufweist, die die direkte und/oder reflektierte Übertragung, ohne vorheriges Eindringen in das streuende Material, des Lichts zwischen einer Lichtquelle und einem Sensor verhindert, und dass das Fenster des Trägers eine Antireflexionsbehandlung vorweist.

6. Vorrichtung nach Anspruch 4 oder 5, **dadurch gekennzeichnet, dass** die N Lichtquellen $SL_i$ polarisiert sind und dass die Richtung des emittierten Strahls einen Brewster-Winkel mit der optischen Achse der M Sensoren $P_j$ bildet.

7. Vorrichtung nach einem der Ansprüche 4 bis 6, **dadurch gekennzeichnet, dass** sie Funkkommunikationsmittel umfasst, um das Ergebnis der Messung periodisch an eine entfernte Vorrichtung zu übertragen.

8. Vorrichtung nach einem der Ansprüche 4 bis 7, **dadurch gekennzeichnet, dass** die Lichtquellen, die Sensoren und die elektronische Schaltung ein Patch bilden, das Mittel zur reversiblen Verbindung mit einem Patienten vorweist.

9. Vorrichtung nach einem der Ansprüche 4 bis 8, **dadurch gekennzeichnet, dass** sie mindestens zwei Paare aus Lichtquelle und empfindlichem Sensor umfasst, wobei ein erstes Paar aus Lichtquelle und empfindlichem Sensor zwischen dem Sensor und der Quelle einen ersten Trennungsabstand definiert und ein zweites Paar aus Lichtquelle und empfindlichem Sensor einen zweiten Trennungsabstand definiert.

10. Vorrichtung nach dem vorstehenden Anspruch, **dadurch gekennzeichnet, dass** die Quellen der beiden Paare beide mit einem Spektrum emittieren, das um eine Wellenlänge zentriert ist, die nahe bei oder größer als eine Absorptionswellenlänge von Wasser ist, oder dass eine der beiden Quellen mit einem Spektrum emittiert, das um eine Wellenlänge zentriert ist, die nahe bei einem Absorptionspeak von Wasser liegt, und die andere Quelle mit einem Spektrum, das um eine Wellenlänge der Nichtabsorption von Wasser zentriert ist, und dass die Quellen der beiden Paare beide mit einem Spektrum emittieren, das um eine Absorptionswellenlänge von 1.450 nm, 1.500 nm oder 1.550 nm zentriert ist.

11. Vorrichtung nach Anspruch 9, **dadurch gekennzeichnet, dass** die Quellen der beiden Paare beide in einer Wellenlänge der Nichtabsorption von Wasser emittieren, wobei die Quellen der beiden Paare beide mit einem Spektrum emittieren, das um 1.300 nm zentriert ist.

12. Vorrichtung nach einem der Ansprüche 9 bis 11, umfassend ein drittes Paar aus Lichtquelle und Sensor, wobei die Lichtquelle in einer Absorptionswellenlänge von Wasser emittiert, die am nächsten zum Sensor angeordnet ist, umfassend einen Sensor und drei Quellen, wobei die drei Quellen jeweils zu drei verschiedenen Paaren aus Quelle und Sensor gehören, oder umfassend zwei Sensoren und zwei Quellen, wobei eine der beiden Quellen in einer Wellenlänge nahe einem Absorptionspeak von Wasser emittiert und die andere Quelle in einer Wellenlänge der fehlenden Absorption von Wasser.

13. Vorrichtung nach Anspruch 10, die zwischen den beiden verschiedenen Quellen eine Reihe von verschiedenen, in einer Reihe angeordneten Sensoren oder ein lineares Photodioden-Array umfasst.

14. Vorrichtung nach einem der Ansprüche 9 bis 13, umfassend einen zentralen oder exzentrischen Sensor und um diesen Sensor herum eine Folge von Lichtemissionsquellen, die um Wellenlängen der Absorption und/oder der fehlenden Absorption von Licht zentriert sind.

15. Vorrichtung nach einem der Ansprüche 9 bis 14, umfassend mindestens eine zentrale oder exzentrische Lichtemissionsquelle, die mit einem Lichtspektrum emittiert, das um eine Wellenlänge der Absorption oder der fehlenden Absorption von Licht zentriert ist, und um diese Quelle herum eine Folge von Sensoren.

16. Vorrichtung nach einem der Ansprüche 4 bis 15, umfassend eine Maske, die physisch zwischen einer Quelle und einem benachbarten Sensor liegt, wobei die Maske um jede Quelle herum einen Tunnel bildet, der das ausgestrahlte Licht in einem bestimmten Bereich konzentriert.

17. Anwendung des Verfahrens und der Vorrichtung nach einem der Ansprüche 1 bis 16 zum Charakterisieren der Wasserkonzentration in einer Lebensmittelmatrix, in einer Pflanze oder in einer Zellulosematrix, wie Papier, Pappe und Holz.

18. Anwendung des Verfahrens und der Vorrichtung nach einem der Ansprüche 1 bis 16 zum Charakterisieren des Feuchtigkeitsgehalts der Haut und/oder zum Charakterisieren der Qualität und/oder der Quantität von Kollagen oder Keratin der Haut.

19. Anwendung des Verfahrens und der Vorrichtung nach einem der Ansprüche 1 bis 16 zum Charakterisieren des Feuchtigkeitsgehalts einer Haarsträhne.

**Claims**

1. A method for measuring the concentration of water in a light-scattering material, the method comprising the following steps:

   - Emission by N light sources (2, 3) $SL_i$ of beams of wavelengths Lambda; onto the material
   - Acquisition by M sensors (4) $P_j$ that are sensitive in at least a portion of said wavelengths Lambda; of signals $S_j$ emitted by the material
   - where M+N≥3

   the method further comprising the steps of:

   - calculating a first item of information $I_{dif}$ representative of the scattering and a second item of information $I_{abs}$ representative of the absorption, according to said signals $S_j$ and an item of numerical information representative of the scattering material on the one hand and said sources $SL_i$ and said sensors $P_j$ on the other hand,
   - calculating said concentration of water using the second item of information $I_{abs}$,

   the method being **characterized in that** the item of numerical information is provided by one or more functions which are determined in a calibration step by acquiring the signals Sj for samples each composed of:

   at least one of an absorbent, a diluent and a scatterer using scattering particles whose shape, size or size distribution make it possible to reproduce the anisotropy of the medium to be studied, and
   an intermediate component selected so as to equalize the refractive index of the diluent and the refractive index of the scattering particles before the addition of the absorbent to the sample.

2. The method for measuring the concentration of water as claimed in claim 1, **characterized in that** the wavelength Lambda; is selected so as to be close to 1450 or 1940, or 970 or 1190 nanometers for the light sources and sensors delivering a signal representative of the scattering at a wavelength corresponding to a water absorption peak.

3. The method for measuring the concentration of water as claimed in claim 1, **characterized in that** the wavelength Lambda; is selected so as to be close to 1050 or 1070, or 1100 or 1300 nanometers for the light sources and sensors delivering the signal representative of the scattering at a wavelength other than a water absorption peak.

4. An apparatus for measuring the concentration of water in a light-scattering material comprising N light sources (2, 3) $SL_i$ emitting in wavelengths Lambda; and M sensors (4) $P_j$ that are sensitive in at least a portion of said wavelengths Lambda;, where N+M≥3, an electronic circuit comprising, for each of said sensors $P_j$, a signal processing means for delivering a signal $S_j$, a processing operation and a computer controlling the processing operation delivering, according to said signals $S_j$ and an item of numerical information representative of the scattering material and of the geometry of said sources SL and of said sensors $P_j$, a first item of information $I_{dif}$ representative of the scattering, a second item of information $I_{abs}$ representative of the absorption and delivering said concentration of water according to the second item of information $I_{abs}$, **characterized in that** the apparatus comprises one or more calibration samples and **in that**

the item of numerical information is provided by one or more functions which are determined in a calibration step by acquiring the signals Sj for the calibration samples each composed of:

at least one of an absorbent, a diluent and a scatterer using scattering particles whose shape, size or size distribution make it possible to reproduce the anisotropy of the medium to be studied, and

an intermediate component selected so as to equalize the refractive index of the diluent and the refractive index of the scattering particles before the addition of the absorbent to the sample.

5. The apparatus as claimed in the preceding claim **characterized in that** said N light sources $SL_i$ and said M sensors $P_j$ are housed in a support opened via a window for contact with the material to be characterized, said support comprising a screen preventing direct and/or reflected transmission, without prior penetration into said scattering material, of the light between a light source and a sensor, and **in that** the window of the support has an anti-reflection treatment.

6. The apparatus as claimed in claim 4 or 5, **characterized in that** said N light sources $SL_i$ are polarized and **in that** the direction of the emitted beam forms Brewster's angle with the optical axis of said M sensors $P_j$.

7. The apparatus as claimed in one of claims 4 to 6, **characterized in that** it comprises radio communication means for periodically transmitting the measurement result to a remote apparatus.

8. The apparatus as claimed in one of claims 4 to 7, **characterized in that** the light sources, the sensors and the electronic circuit constitute a patch that has means for reversible attachment to a patient.

9. The apparatus as claimed in one of claims 4 to 8, **characterized in that** it comprises at least two light source/sensitive sensor pairs; a first light source/sensitive sensor pair defining a first separation distance between the sensor and the source, and a second light source/sensitive sensor pair defining a second separation distance.

10. The apparatus as claimed in the preceding claim, **characterized in that** the sources of the two pairs both emit in a spectrum centered on a wavelength close to or greater than a water absorption wavelength or **in that** one of the two sources emits in a spectrum centered on a wavelength close to a water absorption peak and the other source in a spectrum centered on a wavelength without water absorption, and **in that** the sources of the two pairs both emit in a spectrum centered on an absorption wavelength of 1450 nm, 1500 nm or 1550 nm.

11. The apparatus as claimed in claim 9, **characterized in that** the sources of the two pairs both emit at a wavelength without water absorption, and **in that** the sources of the two pairs both emit in a spectrum centered on 1300 nm.

12. The apparatus as claimed in one of claims 9 to 11, comprising a third light source/sensor pair, the light source emitting in a water absorption wavelength, which is arranged as close as possible to the sensor, comprising a sensor and three sources, the three sources respectively belonging to three discrete source/sensor pairs, or comprising two sensors and two sources, one of the two sources emitting at a wavelength close to a water absorption peak and the other source at a wavelength without water absorption.

13. The apparatus as claimed in claim 10, comprising, between the two discrete sources, a series of discrete sensors arranged in a row or a linear array of photodiodes.

14. The apparatus as claimed in one of claims 9 to 13, comprising a central or off-center sensor and, around this sensor, a succession of light-emitting sources centered on absorption wavelengths and/or wavelengths without light absorption.

15. The apparatus as claimed in one of claims 9 to 14, comprising at least one central or off-center light-emitting source that emits in a spectrum of light centered on an absorption wavelength and/or a wavelength without light absorption and, around this source, a succession of sensors.

16. The apparatus as claimed in one of claims 4 to 15, comprising a mask physically positioned between a source and an adjacent sensor, the mask forming, around each source, a tunnel for concentrating the emitted light into a precise area.

17. The application of the method and apparatus as claimed in any one of claims 1 to 16 to characterize the concentration of water in a food matrix, in a plant, or in a cellulose matrix such as paper, cardboard and wood.

18. The application of the method and apparatus as claimed in any one of claims 1 to 16 to characterize the hydration of the skin and/or to characterize the quality and/or quantity of collagen or keratin in the skin.

19. The application of the method and apparatus as claimed in any one of claims 1 to 16 to characterize the hydration of a lock of hair.

Fig. 1

Fig. 2

Fig. 3

Simulation de l'interaction entre la
lumière et l'échantillon prenant en compte les
paramètres opto-géométriques

$S_1$ : Mesure
émetteur 1

$S_2$ : Mesure
émetteur 2

Détermination des coefficients de diffusion
réduit ($\mu_s$') et d'absorption ($\alpha$)

$S_1 = f(\mu_s') * \exp[-\alpha * L_{eff}(\mu_s')]$
$S_2 = f(\mu_s') * \exp[-\alpha * L_{eff}(\mu_s')]$ $\Big\} \to (\mu_s' ; \alpha)$

Détermination de la concentration $c$
en eau

$\alpha = c * \mu_a \longrightarrow c$

*Calculs*

Fig. 4

2          4

11

Fig. 5

Fig. 6                                                                 Fig. 7

Fig. 8

FIG. 9

FIG. 10

FIG. 11

FIG. 12

| Mesures optiques | Détermination des coefficients de diffusion réduit ($\mu_s'$) et d'absorption ($\alpha$) | Détermination du coefficient de diffusion et de la concentration en eau |
|---|---|---|
| $S_1$<br>$S_i$<br>$S_n$ | $S_1 = f_1(\mu_s', \alpha)$<br>$S_i = f_i(\mu_s', \alpha)$<br>$S_n = f_n(\mu_s', \alpha)$ | $c = \alpha / \mu_a$<br>$\mu_s = \mu_s' / g$ |

FIG. 13

FIG. 14a

FIG. 14b

FIG. 14c

FIG. 15a

Signal LED1 /u.a.

FIG. 15b

Signal LED2 /u.a.

FIG. 15c

Signal LED3 /u.a.

| Exemple | us /cm | c % |
|---|---|---|
| Expérimental | 115 | 66 |
| Mesure | 119 | 66 |

FIG. 16

FIG. 17

FIG. 18a

FIG. 18b

FIG. 19

FIG. 20a

FIG. 20b

FIG. 21a

FIG. 21b

FIG. 21c

FIG. 21e

FIG. 21f

FIG. 21g

FIG. 21d

Mesures optiques

$S_1$

$S_i$

$S_n$

Détermination des coefficients de diffusion ($\mu_s$) et de la concentration en eau (c)

$$S_1 = f_1(\mu_s, c)$$
$$S_i = f_i(\mu_s, c)$$
$$S_n = f_n(\mu_s, c)$$

$\left. \right\}$ $\mu_s, c$

FIG. 22

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8180419 B
- EP 2956049 A
- US 6442408 B
- US 20140016132 A1
- US 2011018468 A1

**Littérature non-brevet citée dans la description**

- **S.ANDREE et al.** *Medical Laser application,* 2011, vol. 26, 109-118
- **R.NACHABÉ et al.** *Journal of Biomédical Optics,* Mai 2010, vol. 15 (3), 037015-1, 037015-10